# EUROPEAN PATENT APPLICATION

(11) **EP 1 722 230 A2**
(43) Date of publication of application: **15.11.2006**
(21) Application number: 06008521.4
(22) Date of filing: 25.04.2006
(51) Int. Cl.: G01N 33/569, G01N 33/50, G01N 33/68, G01N 1/30, C07K 16/28, A61K 39/395, C40B 40/00

(54) **Method for detection of a disease-specific combinatorial molecular pattern motif in a tissue sample of a patient's skin**

(30) Priority: 27.04.2005 US 675148 P
(71) Applicant: MPB MelTec Patent- und Beteiligungsgesellschaft mbH, 39120 Magdeburg (DE); Otto-Von-Guericke-Universität Magdeburg, 39120 Magdeburg (DE)
(72) Inventor: Pommer, Ansgar J., Dr., 39108 Magdeburg (DE); Philipsen, Lars, Dipl.-Ing., 39114 Magdeburg (DE); Bastian, Anja, 39128 Magdeburg (DE); Gollnick, Harald, Prof. Dr., 39130 Magdeburg (DE); Bonnekoh, Bernd, Prof. Dr., 39116 Magdeburg (DE); Böckelmann, Raik, Dr., 39108 Magdeburg (DE); Schubert, Walter, Dr., 39175 Biederitz (DE)
(74) Representative: Patentanwälte Hofstetter, Schurack & Skora

(57) **Abstract**

The invention relates to a method for detection of a disease-specific combinatorial molecular pattern motif in a tissue sample of a patient's skin and/or a tissue sample of skin-neighboured-mucosa, wherein the spatial arrangement of epitopes in the sample is captured for identifying the disease-specific combinatorial molecular pattern motif. The invention moreover relates to compositions and kits comprising antibodies and/or ligands which can be employed for the performance of the method.

## Description

The invention relates to a method for detection of a disease-specific combinatorial molecular pattern motif in a tissue sample of a patient's skin and/or a tissue sample of skin-neighbored-mucosa. The invention moreover relates to compositions and kits comprising antibodies and/or ligands which can be employed for this method.

Such method as well as such compositions and kits can be employed for conducting laboratory tests for identifying psoriasis on the one hand and atopic dermatitis on the other hand.

Psoriasis vulgaris, in a word psoriasis, is a chronic inflammatory skin disease with a substantial socio-economic impact and has generally been hypothesized as a T-cell mediated auto-immune disease with a spectrum of auto-antigens, e.g. keratin peptides, residing in the epidermo-papillary compartment. As an anti-thesis pathogenetic mechanisms mimicking autoimmunity and involving e.g. NK cells have been advocated.

Atopic dermatitis is part of the atopy syndrome which is supposed to be a multifactorial disease cluster decisively governed by genetic as well as environmental factors. Dysregulation of IgE production, TH1/TH2 dysbalances, mutations in the promotor regions of pivotal cytokines like IL-4, IgE autoreactivity as well as hyper-releasability phenomena in mast cells and lymphocytes have been envisioned as mosaic stones in the complex pathogenetic picture of atopic disorders. Moreover, atopic diseases have been subcategorized into intrinsic (non-allergic) and extrinsic (allergic) variants, the latter being driven especially by IgE-mediated allergens from the environment.

Up to now inflammatory dermatoses such as psoriasis and atopic dermatitis could only be discerned or diagnosed by an experienced physician by way of examining the surface of the skin. As the external appearance, however, often does not allow for drawing clear conclusions and also the demarcation against other diseases such as e.g. a lymphoma often proves difficult, diagnostic errors which may involve grave negative consequences for the patient concerned occur regularly. But even if the diseases are correctly diagnosed, in particular as to the atopic dermatitis it is difficult to find an effective method of treatment. As to psoriasis, by contrast, a treatment with a human antibody directed against the epitope CD11a (= CFA1) is known which is usually performed with success.

The invention therefore is based on the object of providing an option for the reliable identification of psoriasis and atopic dermatitis and an effective means for the treatment of atopic dermatitis.

This object according to the invention is solved by way of a method, compositions, kits as well as applications with the features of the independent claims.

Further advantageous variations are defined in the subclaims.

The solution of the object is based on the knowledge that psoriasis and atopic dermatitis can be identified by aid of the so-called Multiple Epitope Ligand Cartography (MELC) robot technology by identifying certain epitopes on the basis of tissue samples. In this way combinational molecular phenotype or pattern (CMP) motifs, specific to the two inflammatory dermatoses in tissue samples could be established. This allows for providing corresponding laboratory tests.

The information resulting from conventional immuno-phenotyping techniques is restricted by a limited resolution with regard to the numbers of epitopes to be detected simultaneously in a given sample or tissue section. These problems are to some extent circumvented by the MELC robot technology described in detail in the patent documents US 6,150,173, US 6,924,115, US 6,974,675, DE 197 09 348, EP 0 810 428, EP 1 136 822, and EP 1 136 823. Its principle involves the sequential automated incubation of a tissue section or cell (blood) smear by various antibodies and/or ligands labeled for example with fluorophores including soft bleaching steps in between. Fluorescence signals are recorded by an automated microscope and camera in a pixel-matrix. Besides the fluorophores also other labelings which allow for a precise detection of the epitopes labeled by the ligands or antibodies bond can be employed. Subsequent binarization and computerized analysis of electronically filed image data allow to detect positive and negative coexpressions of multiple epitopes in a pixel-based manner as so-called CMP's.

This technology is applied to normal and diseased skin tissue surmounting technical obstacles due to organ-specific strong autofluorescence. Skin affected with psoriasis and atopic dermatitis is studied with a library antibodies detecting constitutive and inflammation-related epitopes in comparison to healthy control tissue (n=10 individuals, each).

Some CMP's (or slight variants of them) are extremely common, and appear to define functional regions of the cell or tissue in question. These features can be formally defmed as CMP motifs: sets of CMP's in which: (i) one or more proteins are invariably present (the lead protein(s)); (ii) one or more proteins are never present (absent proteins); and (iii) additional proteins are present in some but not all members of the set (wild-card proteins). Each motif can thus be denoted by a sequence of 1s, 0s, and *s, indicating the lead, absent, and wild card proteins, respectively. By assigning one colour to each (relevant) pixel- or whole cell-CMP motif, a map of the motifs within any given cell or tissue under investigation can be produced, summarizing the protein organization throughout the region termed a toponome map. As protein compartmentalization in cells and tissues determines structure and function, toponome maps make it possible to identify spatially confined functional entities for the first time.

A first CMP-motif with the lead proteins CD45RA, CLA and CD38 is prominently upregulated in psoriasis. According to the invention therefore a method for detecting the psoriasis-specific CMP-motif in a tissue sample of a patient's skin is provided, with the spatial arrangement of the epitopes CD45RA, CLA and CD38 in the tissue sample of a patient's skin being captured for identifying the psoriasis-specific combinatorial molecular pattern motif, "CD45RA *on* / CLA *on* / CD38 *on".* The identification of this CMP-motif allows for a highly reliably statement saying that an examined tissue sample is the sample of a psoriasis patient. Moreover the performance of this method requires a very short period of time, so that the result is immediately available and a quick decision on a subsequent treatment can be used.

Additionally a second CMP-motif has been identified, which is characterized by the lead protein CD30 and absent protein CD7 and which is specifically up-regulated in atopic dermatitis in comparison to psoriasis and normal skin. According to the invention therefore a method is provided, wherein the spatial arrangement of the epitopes CD30 and CD7 in the tissue sample of a patient's skin is captured for identifying the combinatorial molecular pattern motif, "CD30 *on* / CD7 *off*", being specific to atopic dermatitis.

Both motifs are largely confined to the upper dermis, and probably represent subpopulations of disease-relevant, activated T cells. Thus advantageously the tissue sample is a sample of the upper dermis.

One of the proteins upregulated in both conditions is CD11a, which is a subunit of LFA-1, a ligand for CD54 (ICAM-1). According to the invention therefore also a method is provided, wherein the spatial arrangement of the epitope CD11a and a T-cell-specific epitope in the tissue sample of a patient's skin is captured for identifying the third specific combinatorial molecular pattern motif, "CD11a *on* / T-cell specific epitope on", being specific to psoriasis and atopic dermatitis. Because sites containing CD 11a are significantly more likely to contain CD2, CD3, CD4 and CD30 in both diseases, in a further variation of the invention the spatial arrangement of the epitopes CD11a, CD2, CD3, CD4 und CD30 in the tissue sample of a patient's skin is captured for identifying the specific combinatorial molecular pattern motif, "CD11a *on* / CD2 *on*/CD3 *on*/CD4 *on*/CD30 *on ".*

Additionally a fourth CMP-motif has been identified with the lead proteins CD36 and HLA-DR together with the absent protein CD29 whose abundance accurately discriminate between the three skin conditions (P < 0.00018). According to the invention therefore a method is provided, wherein the expression of the combinatorial molecular pattern motif is detected and wherein a maximum expression of this combinatorial molecular pattern is specific to psoriasis, an intermediate expression is specific to atopic dermatitis and a minimum expression is specific to normal skin, wherein the spatial arrangement of the epitopes HLA-DR, CD36 and CD29 in the tissue sample of a patient's skin is captured for identifying the specific combinatorial molecular pattern motif, "HLA-DR *on* / CD36 *on* / CD29 *off".*

A systematic analysis of the remaining molecules identifies an associated motif in which the original lead and absent proteins are joined by the further lead proteins CD58, CD138 and pan cytokeratin, forming a combined motif which is expressed predominantly in keratinocytes from the upper part of the epidermis. In an variation of the invention therefore a method is provided, wherein the spatial arrangement of the further epitopes CD58, CD138 and pan-cytokeratin in the tissue sample is captured for identifying the specific combinatorial molecular pattern motif, "HLA-DR *on* / CD58 *on* / CD138 *on* / CD36 *on* / pan cytokeratin *on* / CD29 *off".*

Advantageously the tissue sample used in the two last-mentioned methods is a sample of the epidermis.

In a further variation of the invention it is provided to capture the spatial arrangement of the epitopes by means of employing in particular labeled antibodies and/or biologics and/or parts of biologics and/or ligands binding specifically to the epitopes. Above all monoclonal antibodies have a very high specificity due to the employment of preparation techniques known to the expert in the field.

A fluorescent dye particularly easy to detect is suitable for labeling. Examples herefor are phycoerythrin or fluorescein. Moreover labeling with quantum dot is possible.

The spatial arrangement of the epitopes may be captured in an advantageous variation of the invention via the sequence of the following method steps: (a) applying a solution to the tissue sample; (b) allowing the solution to take effect and removing the solution; (c) recording an image prior to or after removing the solution; with the solution containing at least one labeled antibody and/or ligand specifically binding to one of the epitopes. This sequence is the core of the already mentioned MELC method which is described in detail in the printed patent specifications US 6,150,173, US 6,924,115, US 6,974,675, DE 197 09 348, EP 0 810 428, EP 1 136 822, and EP 1 136 823 and to which reference is made herewith.

Steps (a) to (c) may also be repeated with at least one further solution which equally contains at least one labeled antibody and/or ligand binding specifically to one of the further epitopes. Moreover possibly subsequent to step (b) and/or step (c) a washing step and/or subsequent to step (c) a bleaching step may be performed. The bleaching step may prove necessary or at least advantageous when employing antibodies and/or ligands coupled to the fluorescent dye.

Particularly preferred for the identification of the first CMP-motif is the employment of at least one antibody which is member of the following group:
- ALB11, an in particular fluorescein-labeled antibody which is directed against CD45RA,
- HECA-452, an in particular fluorescein-labeled antibody which is directed against CLA,
- T 16, an in particular fluorescein-labeled antibody which is directed against CD38.

For the identification of the second CMP-motif at least one antibody can be employed which is member of the following group:
- Ber-H2, an in particular fluorescein-labeled antibody which is directed against CD30,
- 8H8.1, an in particular fluorescein-labeled antibody which is directed against CD7.

Particularly preferred for the identification of the third CMP-motif is the employment of the antibody MHM24, an in particular fluorescein-labeled antibody which is directed against CD11a. Additionally at least one antibody might be employed which is member of the following group:
- 39C1.5, an in particular fluorescein-labeled antibody which is directed against CD2,
- UCT1, an in particular fluorescein-labeled antibody which is directed against CD3,
- Coulter T4, an in particular fluorescein-labeled antibody which is directed against CD4,
- Ber-H2, an in particular fluorescein-labeled antibody which is directed against CD30.

For the detection of the fourth CMP-motif at least one antibody can advantageously be employed which is member of the following group:
- Immu-357, an in particular fluorescein-labeled antibody which is directed against HLA-DR,
- FA6-152, an in particular fluorescein-labeled antibody which is directed against CD36,
- 4B7R, an in particular fluorescein-labeled antibody which is directed against CD29.

Additionally at least one antibody might be employed which is member of the following group:
- AICD58, an in particular fluorescein-labeled antibody which is directed against CD58,
- B-B4, an in particular fluorescein-labeled antibody which is directed against CD138,
- MNF116, an in particular fluorescein-labeled antibody which is directed against pan cytokeratin.

The combination of letters/numbers mentioned designates the clone from which the respective antibody is obtained. Besides the fluorescein labelings of course other labelings with fluorescent dyes or quantum dot are possible, too. The named antibodies have proven particularly suitable in a number of tests.

In a particularly preferred variation of the invention the MELC robot technology is used for the detection of the disease-specific combinatorial molecular pattern motif.

The above mentioned object is also solved by providing compositions or kits, which comprise at least one antibody and/or at least one ligand, wherein the antibodies and/or ligands bind the epitopes of the mentioned CMP-motifs specifically and are coupled with particularly different labelings each. If different labelings are used, the individual antibodies and/or ligands and thus also the epitopes bound through them can be differentiated in the case of a simultaneously performed labeling. By employing the composition according to the invention the time required for the identification of the CMP motif is considerably shortened and the performance of the above method facilitated.

In a kit the individual antibodies and/or ligands are contained in different receptacles, but are still made available to the user simultaneously. When using such a kit it is not necessary that the labelings of the epitopes are performed simultaneously, so that the antibodies and/or biologics and/or parts of a biologics and/or ligands may also be coupled with identical labelings.

In a preferred variation of the invention at least one of the labelings is a fluorescent dye, in particular phycoerythrin or fluorescein, or a quantum dot.

One variation of the invention exhibits a composition or kit, wherein at least one antibody is employed which is a member of the following group:
- ALB11, an in particular fluorescein-labeled antibody which is directed against CD45RA,
- HECA-452, an in particular fluorescein-labeled antibody which is directed against CLA,
- T16, an in particular fluorescein-labeled antibody which is directed against CD 38.

Another variation of the invention exhibits a composition or kit, wherein at least one antibody is employed which is a member of the following group:
- Ber-H2, an in particular fluorescein-labeled antibody which is directed against CD 30,
- 8H8.1, an in particular fluorescein-labeled antibody which is directed against CD 7.

A further variation of the invention exhibits a composition or kit comprising the antibody MHM24, an in particular fluorescein-labeled antibody which is directed against CD11 a.

Additionally the composition or kit might comprise at least one antibody which is member of the following group:
- 39C1.5, an in particular fluorescein-labeled antibody which is directed against CD2,
- UCT1, an in particular fluorescein-labeled antibody which is directed against CD3,
- Coulter T4, an in particular fluorescein-labeled antibody which is directed against CD4,
- Ber-H2, an in particular fluorescein-labeled antibody which is directed against CD30.

Another composition or kit according to the invention might comprise at least one antibody which is member of the following group:
- Immu-357, an in particular fluorescein-labeled antibody which is directed against HLA-DR,
- FA6-152, an in particular fluorescein-labeled antibody which is directed against CD36,
- 4B7R, an in particular fluorescein-labeled antibody which is directed against CD29.

Additionally this composition or kit might comprise at least one antibody which is member of the following group:
- AICD58, an in particular fluorescein-labeled antibody which is directed against CD58,
- B-B4, an in particular fluorescein-labeled antibody which is directed against CD138,
- MNF116, an in particular fluorescein-labeled antibody which is directed against pan cytokeratin.

As already stated above, all of the mentioned antibodies have proven particularly favourable in a number of tests.

The described compositions or kits are suitable for being employed in the diagnosis, especially in the diagnoses of skin diseases as part of a laboratory test and above all in the diagnosis of psoriasis and atopic dermatitis.

According to the invention also a biochip is provided, wherein on one surface of the chip at least ligand and/or at least one antibody and/or at least one biologics and/or at least one part of a biologics binding specifically to a specific combinatorial molecular pattern motif are coupled in such a way that their bondability to the motif is sustained.

The above-mentioned object according to the invention is also solved by the use of the human antibody efalizumab directed against the CD11a for preparing a remedy for treating atopic dermatitis. This is because it could be shown that CD11a also plays a central role with the atopic dermatitis during the intrusion process of the T-cells into the dermis, so that this process is prevented by inhibiting the CD11a by aid of the corresponding human antibody. In this way the change of the skin typical of atopic dermatitis can be prevented.

As documented according to the invention the MELC robot technology is suitable for identification of a combinatorial molecular pattern as anatomical, biochemical, pathogenetic, diagnostic and/or therapeutic toponome leads in skin tissue and tissue of skin-neighboured-mucosa for the discrimination of healthy and disease-dependent states.

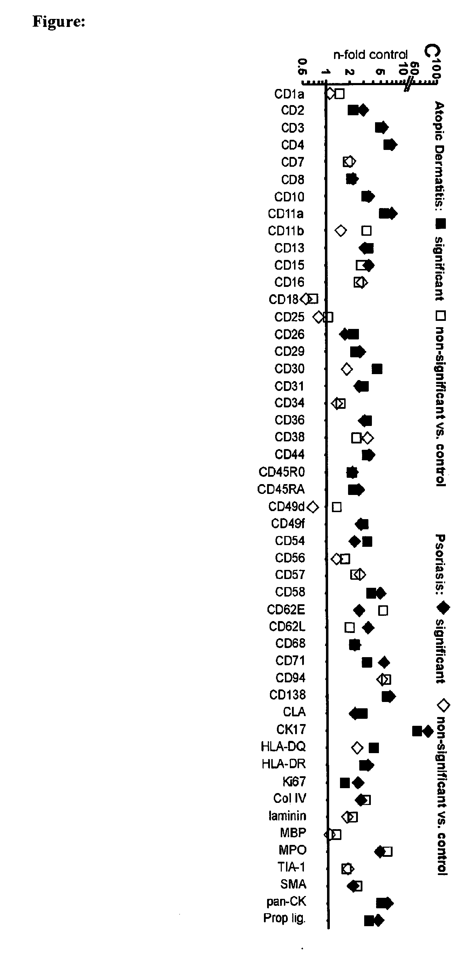

Additional features and advantages of the invention derive from the following description of several embodiments partially being based on the Figure.

The Figure shows a graph depicting the average frequency of 49 molecules across the entire section in biopsies from patients with psoriasis (diamonds) and atopic dermatitis (squares) relative to frequency in biopsies from healthy controls. Note the scarcity of statistically significant differences (filled symbols, significant difference; open symbols, insignificant difference).

In a first embodiment skin biopsies from a patient suffering from psoriasis vulgaris are analyzed and compared to skin biopsies from a patient suffering from atopic dermatitis and to skin biopsies from healthy people. In detail, the psoriasis patients comprise 5 male and female individuals, each. Psoriasis type 1 and 2 are represented by 6 and 4 patients, respectively, 2 patients additionally affected by psoriatic arthritis. Among the patients with atopic dermatitis (5 males and females, each) the average total serum IgE level is significantly increased by 5,938 ± 6,135 kU/ml (range from 55.6 to 16,997 kU/ml) and the serum eosinophilic cationic protein by 47.3 ± 17.3 (15.8 - 68.0 µg/l). Biopsies of representative skin lesions are taken at the time of inpatient admission because of severe disease deterioration after a wash-out period of 4 or 2 weeks for any specific systemic-/UV- or topical treatment. Normal skin donors (4 males, 6 females) are not affected by any inflammatory skin or system disease, nor do they use any immuno-suppressive systemic medication. The age distribution is characterized by a range from 22 to 84 years with an average at 51.0± 18.0 years.

Punch biopsies of 6 mm diameter are taken from affected and normal skin of patients and donors in local anesthesia using a 1% prilocaine hydrochloride solution.

Since the methods according to the invention are preferably only performed in the laboratory, the sample-taking preferably is not part of the method according to the invention.

Freshly taken skin biopsies are snap frozen in liquid nitrogen. In brief, the tissue is placed in frozen specimen-embedding medium at room temperature. The specimen is transferred into liquid isopentane pre-cooled with liquid nitrogen and frozen for 60 seconds. Storage is performed at -80 °C.

Tissue sections of 5 µm thicknesses are prepared at -25°C using a cryotome (Shandon), fixed in acetone at -20°C for 10 min and stored at -20°C for several days or -80°C for longer time intervals until use. Before use, all samples are rehydrated in PBS at room temperature, incubated with normal goat serum for 30 min, and washed again in PBS.

Aspects of MELC technology have been described above. In brief, a slide with a given specimen is placed on the stage of an inverted wide-field fluorescence microscope (Leica or Zeiss) equipped with fluorescence filters for FITC and PE. Fluorochrome-labelled antibodies and wash solutions are added and removed robotically under temperature control, avoiding any displacement of the sample and objective. In each cycle, a pair of antibodies is added; phase contrast and fluorescence images are acquired by a high-sensitivity cooled CCD camera; the sample is washed ten times with PBS and bleached at the excitation wavelengths for 10 min; and post-bleaching phase contrast and fluorescence images are acquired. The cycle is then repeated with the next set of antibodies. Different developmental stages of the MELC system are progressively automated by using proprietary software. The motor-controlled stage of the microscope is used to record several visual fields in each cycle, which are fused to generate panoramic images.

Fluorescence images produced by each antibody are aligned pixel-wise using the phase contrast images. Background and illumination faults are then removed by flat-field correction before excluding artefacts by a mask-setting process. Fluorescence signals are set to 0 (= below threshold) or 1 (= above threshold), using an automated threshold setting method validated by human experts. The binarized images are then combined to form a list of CMPs representing the proteins expressed in each pixel. To analyse these further the 'MotifFinder' and 'MotifAnalyzer' software packages are developed to search for and visualize CMP motifs. CMPs and CMP motifs are then colour-coded and superimposed on the corresponding biological structures to create co-localization and toponome maps.

The MotifFinder algorithm is used to search for CMPs or CMP motifs distinguishing biological samples collected under different conditions (e.g. samples from patients as opposed to controls or samples from different biological compartments). The search for motifs is performed by examining the distribution of all combinations of two molecules, followed by all combinations of three molecules, and so on up to a limit imposed by computational resources. The significance of motif frequencies is assessed by using the Wilcoxon rank-sum test or Student t-test.

Three-dimensional imaging of MELC runs is performed by generating and visualizing z-stack raw images for each antibody signal from top to bottom of a sample, followed by the steps of: deconvoluting these images using a standard algorithm working with a specific point spread function; setting thresholds for each antibody signal from each optical plane as before; overlaying all binarized images to construct large scale protein colocalization maps using a MATLAB algorithm; and constructing three-dimensional toponome maps in the same way as is done for two dimensions. The latter two visualization steps are performed by using algorithms provided by IMARIS software packages.

In this first embodiment of the invention the location of 49 molecules shown in the Table are analysed chosen to detect a) cell types (keratinocytes, dendritic cells, macrophages, T lymphocytes, NK cells, neutrophil and eosinophil granulocytes, endothelial cells); b) extracellular matrix components (collagen type IV, laminin); and c) inflammatory cell functions (cell activation, proliferation, adhesion). In the Table the library of fluorophorelabeled antibodies as established for the MELC analysis of skin tissue is depicted, wherein the antibodies for CD1a and TIA-1 are marked by phycoerythrin and all remaining are marked by fluorescein. The sequel order of the antibodies within the MELC run is designated by their position in the Table. The sources are the following: a - Progen, b - DAKO, c - Immunotech, d - Ancell, e - Beckman Coulter, f- Sigma, g - Serotec, h - Biozol, j - Zymed, k - Euroclone, m - An der Grub, n - Hölzel, p - Becton Dickinson. The frequency of pixels positive for each molecule in each tissue compartment (such as epidermis or dermis) is normalized to a horizontal width of 100 µm so as to avoid being influenced by the vertical stratification of epithelial tissue, and tissue compartments are distinguished by positive and negative masks defined largely by means of a pan-cytokeratin antibody (results not shown). As shown in the Figure, 26 of the molecules are significantly upregulated in both diseases, although of these, three are significantly more abundant in psoriasis than in atopic dermatitis. Six further molecules are significantly upregulated in psoriasis alone, and two in atopic dermatitis.

Candidate keratinocyte stem cells have the phenotype Ki67-/CD71-/pan-CK⁺/CD29⁺/CD49d⁺ but it has not so far been possible to map the distribution of cells defined by such a complex phenotype in tissue sections. Such cells can however be readily identified in MELC co-localization maps. The number of such cells is somewhat reduced in atopic dermatitis (24±12% of basal epidermal cells) and significantly reduced in psoriasis (11±7%) compared to healthy controls (34±7%), possibly reflecting a disease-induced increase in the proliferative cell fraction.

One of the proteins upregulated in both conditions is CD11a, which is a subunit of LFA-1, a ligand for CD54 (ICAM-1). LFA-1:CD54 interactions are critical for T cell activation, facilitating translocation of T cells from the vasculature to sites of inflammation, and a monoclonal antibody targeting CD11a (efalizumab) has recently been approved in the EU for the treatment of psoriasis. Therefore the proteins clustered with CD11a in both diseases have been investigated more thoroughly. Sites containing CD11a are significantly more likely to contain CD54 in atopic dermatitis, and to contain CD2, CD3, CD4 and CD30 in both diseases. In the epidermis, they are also more likely to contain CD8 in both diseases, but in the dermis, this is only true in atopic dermatitis. By contrast, healthy skin is characterized by a family of motifs lacking CD11a. Despite the slight differences between the motifs characterizing the two conditions, these results prove that efalizumab-like drugs are also beneficial in atopic dermatitis.

In a further embodiment it is attempted to find other CMP motifs characteristic of each disease, focussing on 20 molecules specific to T and NK cells and analysing all combinations of up to 8 molecules. A motif with the lead proteins CD45RA, CLA and CD38 is prominently upregulated in psoriasis, and one with the lead protein CD30 and absent protein CD7 is similarly up-regulated in atopic dermatitis (data not shown). Both motifs are largely confined to the upper dermis, and probably represent subpopulations of disease-relevant, activated T cells. CLA, a lead protein in the motif characteristic of psoriasis, is an E-selectin ligand expressed on skin lymphocytes, and its presence together with CD45RA (a protein induced on T cells undergoing the naive to memory transition) points to the existence of a hitherto unrecognized subset of skin-based T cells with a role in psoriasis. The most frequent flanking epitopes are HLA-DR (77%) for the psoriasis-related motif core "CD45RA *on* / CLA *on* / CD38 *on ",* and CD4 (74%), CD54 (48%), HLA-DQ (43%), CD8 (28%) und HLA-DR (28%) for the atopic dermatitis-related motif core "CD30 *on* / CD7 *off".*

To increase the range of molecular combinations being analysed, the search is repeated in a further embodiment of the invention, working with all 49 molecules shown in the Table and considering all combinations of up to 4 molecules. As a first step psoriasis and atopic dermatitis are compared with a second step comparing the resulting significantly different CMPs with the healthy control condition, using Wilcoxon rank-sum test. This identifies a motif with the lead proteins CD36 and HLA-DR together with the absent protein CD29 whose abundance accurately discriminates between the three skin conditions (P < 0.00018). A systematic analysis of the remaining molecules identifies an associated motif in which the original lead and absent proteins are joined by the further lead proteins CD58, CD138 and pan-CK, forming a combined motif which is expressed predominantly in keratinocytes from the upper part of the epidermis. Similarly, the discriminatory motif for psoriasis identified by means of a broader search strategy contains markers characteristic of keratinocytes (pan-CK⁺), the suprabasal epidermal layer (CD29⁻), antigen presenting cells (CD58⁺) and activated keratinocytes (HLA-DR⁺ and CD138⁺), perhaps representing a hallmark of the hyper-activated suprabasal keratinocyte islands typical of the condition. Wild cards are observed for CD26, CD44, CD49d, CD71 and CK17. Thereby, wild cards or negative expressions are possible options for the remaining epitopes. When the CMP motif is superimposed with CD1a, its predominant non-Langerhans cell origin is confirmed.

**Table:**

| **Nr./ No.** | **Epitop/ Epitope** | **Klon/ Clone** | **Verdünnung/ Dilution** | **Nr./ No.** | **Epftop/ Epitope** | **Klon/ Clone** | **Verdünnung/ Dilution** |
|---|---|---|---|---|---|---|---|
| 1 | CK17^{a} | Ks17.E3 | 1:10 | 25 | CD29^{h} | 4B7R | 1:10 |
| 2 | CD1a^{b} | NA1/34 | 1:100 | 26 | CD18^{h} | YFC 118.3 | 1:10 |
| 3 | CD2^{c} | 39C1.5 | 1:10 | 27 | CD49d^{h} | 44H6 | 1:50 |
| 4 | CD38^{c} | T16 | 1:10 | 28 | CD44^{c} | J-173 | 1:200 |
| 5 | CD16^{c} | 3G8 | 1:10 | 29 | CD49f^{h} | 4F10 | 1:10 |
| 6 | CD62L^{c} | SK11 | 1:10 | 30 | CD10^{c} | ALB2 | 1:10 |
| 7 | CD25^{d} | 7G7B6 | 1:100 | 31 | Ki67^{j} | 7B11 | 1:80 |
| 8 | CD62E^{d} | HAE-1 f | 1:100 | 32 | CD45R0^{k} | UCHL1 | 1:50 |
| 9 | CD4^{e} | Coulter T4 | 1:10 | 33 | CD34^{b} | QBEnd10 | 1:100 |
| 10 | CD8^{c} | B9.11 | 1:10 | 34 | MPO^{m} | H-43-5 | 1:50 |
| 11 | BLA-DR^{c} | Immu-357 | 1:50 | 35 | TIA-1^{c} | 2G9 | 1:20 |
| 12 | HLA-DQ' | HK19 | 1:10 | 36 | lamininⁿ | - | 1:50 |
| 13 | CD26^{c} | L272 | 1:10 | 37 | CD30^{b} | Ber-H2 | 1:10 |
| 14 | CD45RA^{c} | ALB11 | 1:10 | 38 | CD68^{b} | KP1 | 1:100 |
| 15 | CD57^{c} | NC1 | 1:10 | 39 | CD31^{d} | 158-2B3 | 1:600 |
| 16 | CD54^{c} | 84H10 | 1:10 | 40 | CD94^{d} | HP-3D9 | 1:60 |
| 17 | CD56^{c} | NCAM16.2 | 1:10 | 41 | MBPⁿ | - | 1:50 |
| 18 | CD7^{c} | 8H8.1 | 1:10 | 42 | CD11a^{b} | MHM24 | 1:50 |
| 19 | CD58^{c} | AICD58 | 1:10 | 43 | CLAP | HECA-452 | 1:10 |
| 20 | CD138^{g} | B-B4 | 1:200 | 44 | SMA' | 1A4 | 1:100 |
| 21 | CD13^{c} | SJ1D1 | 1:10 | 45 | pan-CK^{b} | MNF116 | 1:10 |
| 22 | CD71^{c} | YDJ:1.2.2 | 1:50 | 46 | CD3^{c} | UCT1 | 1:10 |
| 23 | CD11b^{c} | 44 | 1:10 | 47 | col IVⁿ | - | 1:1600 |
| 24 | CD36^{c} | FA6-152 | 1:10 | 48 | CD15^{d} | AHN1.1 | 1:40 |

| | | | | | | | |
|---|---|---|---|---|---|---|---|
| No. 49: Prop lig. (nucleic acids) | | | | | | | |

The sequel order of the antibodies within the MELK run is designated by their position in the table.
Sources: a - Progen, b - DAKO, c - Immunotech, d - Ancell, e - Beckman Coulter, f - Sigma, g - Serotec, h - Biozol, j - Zymed, k - Euroclone, m - An der Grub, n - Hölzel, p - Becton Dickinson.
CK = Cytokeratin, MPO = Myeloperoxidase, MBP = Major Basic Protein, SMA = Smooth Muscle Actin, col = Collagen

In a further embodiment of the invention only one tissue sample of a potential psoriasis patient is examined in the above-described manner. Additionally only these antibodies of the Table are employed, which bind specifically to at least one of the psoriasis-specific CMP-motifs "CD45RA *on* / CLA *on* / CD38 on", "CD11a *on* / T-cell specific epitope on", "CD11a *on* / CD2 *on*/ CD3 *on*/ CD4 *on*/ CD30 *on" ,* "HLA-DR *on* / CD36 *on* / CD29 *off"* in a maximum expression and "HLA-DR *on* / CD58 *on* / CD138 *on* / CD36 *on* / pan cytokeratin *on* / CD29 *off"* in a maximum expression, as in this embodiment of the invention at least one of the CMP motifs specific to psoriasis is only identified and not as in the first embodiment detected to start with. The thereby rendered simplification of the method performed in using all the antibodies listed in the Table results in an enormous time gain. The positive or negative identification of the relevant epitopes confirms the assumption that the patient the tissue sample of whom has been examined in deed is affected by psoriasis. In this embodiment the antibodies are applied in the dilutions documented in the Table.

In a further embodiment of the invention only one tissue sample of a potential atopic dermatitis patient is examined in the above-described manner. Additionally only these antibodies of the Table are employed, which bind specifically at least one of the CMP-motifs specific to atopic dermatitis "CD30 *on* / CD7 *off",* "CD11a *on* / T-cell specific epitope *on",* "CD11a *on* / CD2 *on*/ CD3 *on*/ CD4 *on*/ CD30 *on*" , "HLA-DR *on* / CD36 *on* / CD29 *off*" in an intermediate expression and "HLA-DR *on* / CD58 *on* / CD138 *on* / CD36 *on* / pan cytokeratin *on* / CD29 *off"* in an intermediate expression. The positive or negative identification of the relevant epitopes confirms the assumption that the patient the tissue sample of whom has been examined in deed is affected by atopic dermatitis. Also in these embodiments the antibodies are applied in the dilutions documented in the Table.

In a further embodiment of the invention the embodiment listed as first embodiment is modified in such a way that a tissue sample is merely incubated with antibodies and/or ligands which are directed against such epitopes that are indicated in the Figure as significantly increased or reduced in psoriasis or atopic dermatitis.

The antibodies serving for the identification of certain disease-specific epitopes may also be combined in a corresponding antibody composition or be comprised as kit.

## Claims

1. Method for detection of a disease-specific combinatorial molecular pattern motif in a tissue sample of a patient's skin and/or a tissue sample of skin-neighboured-mucosa, wherein the spatial arrangement of epitopes in the sample is captured for identifying the disease-specific combinatorial molecular pattern motif

2. Method according to claim 1, **characterized in that** the spatial arrangement of the epitopes CD45RA, CLA and CD38 in the tissue sample of a patient's skin is captured for identifying the psoriasis-specific combinatorial molecular pattern motif, "CD45RA *on* / CLA *on* / CD38 *on".*

3. Method according to claim 1, **characterized in that** the spatial arrangement of the epitopes CD30 and CD7 in the tissue sample of a patient's skin is captured for identifying the combinatorial molecular pattern motif, "CD30 *on* / CD7 *off",* being specific to atopic dermatitis.

4. Method according to one of the claims 2 or 3, **characterized in that** the tissue sample is a sample of the upper dermis.

5. Method according to claim 1, **characterized in that** the spatial arrangement of the epitope CD11a and a T-cell-specific epitope in the tissue sample of a patient's skin is captured for identifying the specific combinatorial molecular pattern motif, "CD11a *on* / T-cell specific epitope on", being specific to psoriasis and atopic dermatitis.

6. Method according to claim 5, **characterized in that** the spatial arrangement of the epitopes CD11a, CD2, CD3, CD4 und CD30 in the tissue sample of a patient's skin is captured for identifying the specific combinatorial molecular pattern motif, "CD11a *on* / CD2 *on*/ CD3 *on*/ CD4 *on*/ CD30 *on*".

7. Method according to claim 1, **characterized in that** an expression of the combinatorial molecular pattern motif is detected, wherein a maximum expression of this combinatorial molecular pattern is specific to psoriasis, an intermediate expression is specific to atopic dermatitis and a minimum expression is specific to normal skin, wherein the spatial arrangement of the epitopes HLA-DR, CD36 and CD29 in the tissue sample of a patient's skin is captured for identifying the specific combinatorial molecular pattern motif, "HLA-DR *on* / CD36 *on* / CD29 *off".*

8. Method according to claim 7, **characterized in that** the spatial arrangement of the further epitopes CD58, CD138 and pan-cytokeratin in the tissue sample is captured for identifying the specific combinatorial molecular pattern motif, "HLA-DR on / CD58 *on* / CD138 *on* / CD36 *on* / pan cytokeratin *on* / CD29 *off".*

9. Method according to one of the claims 7 or 8, **characterized in that** the tissue sample is a sample of the epidermis.

10. Method according to one of the previous claims, **characterized in that** the tissue sample is a tissue section.

11. Method according to one of the previous claims, **characterized in that** the spatial arrangement of the epitopes is captured by means of employing particularly labeled antibodies and/or biologics and/or parts of biologics and/or ligands binding specifically to the epitopes.

12. Method according to claim 11, **characterized in that** at least one of the labelings is a fluorescent dye, in particular phycoerythrin or fluorescein, or quantum dot.

13. Method according to one of the previous claims, **characterized in that** the spatial arrangement of the epitopes is captured by the sequence of the following method steps:
(a) applying a solution to the tissue sample;
(b) allowing the solution to take effect and removing the solution;
(c) recording an image prior to or after removing the solution;
wherein the solution contains at least one labeled antibody and/or ligand binding specifically to at least one of the epitopes.

14. Method according to claim 13, **characterized in that** steps (a) to (c) are repeated with at least one further solution which also contains at least one labeled antibody and/or ligand specifically binding to at least one of the further epitopes, and **in that** possibly subsequent to step (b) and/or (c) a washing step and/or subsequent to step (c) a bleaching step is performed.

15. Method according to claims 2 and 11, **characterized in that** at least one antibody is employed which is member of the following group:
- ALB11, an in particular fluorescein-labeled antibody which is directed against CD45RA,
- HECA-452, an in particular fluorescein-labeled antibody which is directed against CLA,
- T16, an in particular fluorescein-labeled antibody which is directed against CD 38.

16. Method according to claims 3 and 11, **characterized in that** at least one antibody is employed which is member of the following group:
- Ber-H2, an in particular fluorescein-labeled antibody which is directed against CD 30,
- 8H8.1, an in particular fluorescein-labeled antibody which is directed against CD 7.

17. Method according to claims 5 and 11, **characterized in that** the antibody MHM24 is employed, an in particular fluorescein-labeled antibody which is directed against CD11a.

18. Method according to claims 6 and 11, **characterized in that** at least one antibody is employed which is member of the following group:
- MHM24, an in particular fluorescein-labeled antibody which is directed against CD11a,
- 39C1.5, an in particular fluorescein-labeled antibody which is directed against CD2,
- UCT1, an in particular fluorescein-labeled antibody which is directed against CD3,
- Coulter T4, an in particular fluorescein-labeled antibody which is directed against CD4,
- Ber-H2, an in particular fluorescein-labeled antibody which is directed against CD30.

19. Method according to claims 7 and 11, **characterized in that** at least one antibody is employed which is member of the following group:
- Immu-357, an in particular fluorescein-labeled antibody which is directed against HLA-DR,
- FA6-152, an in particular fluorescein-labeled antibody which is directed against CD36,
- 4B7R, an in particular fluorescein-labeled antibody which is directed against CD29.

20. Method according to claims 8 and 11, **characterized in that** at least one antibody is employed which is member of the following group:
- Immu-357, an in particular fluorescein-labeled antibody which is directed against HLA-DR,
- FA6-152, an in particular fluorescein-labeled antibody which is directed against CD36,
- 4B7R, an in particular fluorescein-labeled antibody which is directed against CD29,
- AICD58, an in particular fluorescein-labeled antibody which is directed against CD58,
- B-B4, an in particular fluorescein-labeled antibody which is directed against CD138,
- MNF116, an in particular fluorescein-labeled antibody which is directed against pan cytokeratin.

21. Method according to one of the previous claims, **characterized in that** the MELC robot technology is used for the detection of the disease-specific combinatorial molecular pattern motif.

22. Composition or kit comprising at least one antibody and/or at least one ligand binding the epitopes CD45RA, CLA and CD38 specifically and being coupled each with in particular different labelings.

23. Composition or kit comprising at least one antibody and/or at least one ligand binding the epitopes CD30 and CD7 specifically and being coupled each with in particular different labelings.

24. Composition or kit comprising at least one antibody and/or at least one ligand binding the epitope CD11a and a T-cell-specific epitope specifically and being coupled each with in particular different labelings.

25. Composition or kit comprising at least one antibody and/or at least one ligand binding the epitopes CD11a, CD2, CD3, CD4 und CD30 specifically and being coupled each with in particular different labelings.

26. Composition or kit comprising at least one antibody and/or at least one ligand binding the epitopes HLA-DR, CD36 and CD29 specifically and being coupled each with in particular different labelings.

27. Composition or kit comprising at least one antibody and/or at least one ligand binding the epitopes HLA-DR, CD36, CD29, CD58, CD138 and pan-cytokeratin specifically and being coupled each with in particular different labelings.

28. Composition or kit according to one of the claims 22 to 27, **characterized in that** at least one of the labelings is a fluorescent dye, in particular phycoerythrin or fluorescein, or a quantum dot.

29. Composition or kit according to claim 22, **characterized in that** at least one antibody is employed which is a member of the following group:
- ALB11, an in particular fluorescein-labeled antibody which is directed against CD45RA,
- HECA-452, an in particular fluorescein-labeled antibody which is directed against CLA,
- T16, an in particular fluorescein-labeled antibody which is directed against CD 38.

30. Composition or kit according to claim 23, **characterized in that** at least one antibody is employed which is a member of the following group:
- Ber-H2, an in particular fluorescein-labeled antibody which is directed against CD 30,
- 8H8.1, an in particular fluorescein-labeled antibody which is directed against CD 7.

31. Composition or kit according to claim 24, **characterized in that** the composition or kit comprises the antibody MHM24, an in particular fluorescein-labeled antibody which is directed against CD 11 a.

32. Composition or kit according to claim 25, **characterized in that** at least one antibody is employed which is member of the following group:
- MHM24, an in particular fluorescein-labeled antibody which is directed against CD11a,
- 39C1.5, an in particular fluorescein-labeled antibody which is directed against CD2,
- UCT1, an in particular fluorescein-labeled antibody which is directed against CD3,
- Coulter T4, an in particular fluorescein-labeled antibody which is directed against CD4,
- Ber-H2, an in particular fluorescein-labeled antibody which is directed against CD30.

33. Composition or kit according to claim 26, **characterized in that** at least one antibody is employed which is member of the following group:
- Immu-357, an in particular fluorescein-labeled antibody which is directed against HLA-DR,
- FA6-152, an in particular fluorescein-labeled antibody which is directed against CD36,
- 4B7R, an in particular fluorescein-labeled antibody which is directed against CD29.

34. Composition or kit according to claim 27, **characterized in that** at least one antibody is employed which is member of the following group:
- Immu-357, an in particular fluorescein-labeled antibody which is directed against HLA-DR,
- FA6-152, an in particular fluorescein-labeled antibody which is directed against CD36,
- 4B7R, an in particular fluorescein-labeled antibody which is directed against CD29,
- AICD58, an in particular fluorescein-labeled antibody which is directed against CD58,
- B-B4, an in particular fluorescein-labeled antibody which is directed against CD138,
- MNF116, an in particular fluorescein-labeled antibody which is directed against pan cytokeratin.

35. Composition or kit according to one of claims 22 to 34 for the use in the diagnosis.

36. Biochip for use in the method of claim 2, 3, 5, 6, 7 or 8, wherein on one surface of the chip at least one ligand and/or at least one antibody and/or at least one biologics and/or at least one part of a biologics binding specifically to the specific combinatorial molecular pattern motif are coupled in such a way that their bondability to the motif is sustained.

37. Use of the composition or kit according to one of claims 22 to 34 for preparing a means of the diagnosis of psoriasis and/or atopic dermatitis.

38. Use of the human antibody efalizumab directed against CD11a for preparing a remedy for the treatment of atopic dermatitis.

39. Use of the MELC robot technology for identification of a combinatorial molecular pattern as anatomical, biochemical, pathogenetic, diagnostic and/or therapeutic toponome leads in skin tissue and tissue of skin-neighbored-mucosa for the discrimination of healthy and disease-dependent states.
